# EUROPÄISCHE PATENTSCHRIFT

(11) **EP 0 618 231 B1**
(45) Veröffentlichungstag und Bekanntmachung des Hinweises auf die Patenterteilung: **02.06.1999**
(21) Anmeldenummer: 94104675.7
(22) Anmeldetag: 24.03.1994
(51) Int. Cl.: C07K 19/00, G01N 33/531

(54) **Immunologisch aktive Konjugate und ein Verfahren zu ihrer Herstellung**
Immunologically active conjugates and a method for their preparation
Conjugués immunologiquement actifs et méthode pour la préparation des-dits

(30) Priorität: 29.03.1993 DE 4310142
(43) Veröffentlichungstag der Anmeldung: 05.10.1994
(73) Patentinhaber: Roche Diagnostics GmbH, 68298 Mannheim (DE)
(72) Erfinder: Markert-Hahn, Christine, Dr., D-82402 Seeshaupt (DE); Ofenloch-Haehnle, Beatus, Dr., D-82407 Wielenbach (DE); Hoess, Eva, Dr., D-82319 Starnberg (DE); Huber, Erasmus, Dr., D-86923 Finning (DE)

(56) Entgegenhaltungen:
- EP-A- 0 446 071
- WO-A-92/22583
- 'ImmunoTechnology Catalog & Handbook' 1990 , PIERCE CHEMICAL COMPANY pages E-4 to E-10 and C-20 to C-27

## Beschreibung

Gegenstand der Erfindung sind immunolgisch aktive Konjugate aus Haptenen, Proteinen und Polymeren, welche stabil gebunden vorliegen.

Für die Bindung von Proteinen, Haptenen und Polymeren aneinander sind eine Vielzahl von Verfahren beschrieben. Üblicherweise werden zwei derartige Substanzen über bifunktionelle Linker kovalent gekoppelt. Ein Überblick der Methoden ist zu finden in M. Brinkley, Bioconjugate Chem. 3 (1992) 2 - 13 und Wong, Chemistry of protein conjugating and cross-linking, CRC press 1991. Besonders bevorzugt ist die Kopplung über die Bildung einer Thioetherbindung (MH-SH-Kopplung) aus einer Maleinimidylgruppe und einer Thiolgruppe.

Die Bindung über derartige Linker ist jedoch häufig nicht stabil, und bei Lagerung in flüssigem und lyophilisiertem Zustand erfolgt ein langsamer Zerfall dieser Konjugate. Dies ist insbesondere bei der Verwendung der Konjugate in Immunoassays nachteilig, da hierdurch Empfindlichkeit, Genauigkeit und Richtigkeit des Meßergebnisses beeinflußt werden.

Die Aufgabe der vorliegenden Erfindung war es daher, stabile, immunologisch aktive Konjugate bereitzustellen, die eine wesentlich stabilere Bindung der zu konjugierenden Substanzen besitzen, als die aus dem Stand der Technik bekannten Linker.

Die Aufgabe wird gelöst durch Konjugate der allgemeinen Formel

A-L₁-L₂-B

in der A und B die zu koppelnden Substanzen, ausgewählt aus der Gruppe der Haptene, Proteine (z. B. Antikörper, Enzyme, wie POD, β-Galactosidase oder alkalische Phosphatase) Polysaccharide (wie z. B. Dextran) lösliche Polystyrole (z. B. Latices) oder Peptide (z. B. ED, Untereinheit der β-Galactosidase), L₁ und L₂ trivalente Linker darstellen und L₁ und L₂ über zwei Thioetherbindungen miteinander verbunden sind.

Die Linker L₁ und L₂ sind chemische Verbindungen, die aus drei, über ein C-Atom gekoppelten, Seitenarmen (Spacer) bestehen. Zwei dieser Seitenarme des Linkers L₁ enthalten jeweils eine Maleinimidogruppe und zwei der Seitenarme des Linkers L₂ enthalten jeweils eine Thiolfunktion. Zur Kopplung der Substanzen A und B über die Linker L₁ und L₂ werden die beiden Maleinimidogruppen und die Thiolfunktion durch SH-Gruppen-Addition an Maleinimid zu zwei Thioetherbindungen verknüpft.

Die erfindungsgemäß verwendeten Linker enthalten außer den beiden Maleinimido- bzw. Thiolfunktionen eine weitere Funktion, die zur Bindung der Linker an die zu koppelnden Substanzen A und B (beispielsweise Haptene, Proteine, Polymere oder Peptide) geeignet sind.

Zur Kopplung der Substanzen A und B werden zwei homobidentale trifunktionelle Linker verwendet, von denen der erste Linker zwei Maleinimidogruppen und eine Hydroxysuccinimid- oder Carbodiimidazolylfunktion trägt und der zweite Linker zwei Thiolgruppen bzw. über Acylgruppen geschützte Thiolgruppen sowie ebenfalls eine Hydroxysuccinimid- bzw. Carbodiimidazolyl-funktion trägt.

Zur Herstellung der erfindungsgemäßen Konjugate werden die zu koppelnden Substanzen, über Hydroxysuccinimid- bzw. Carbodiimidazolylfunktionen, jeweils an einen der beiden homobidentalen trifunktionellen Linker gekoppelt und in einem zweiten Schritt die Maleinimidofunktionen mit den Thiolfunktionen zu zwei Thioetherbindungen umgesetzt.

Die hierbei verwendeten Verfahren sind dem Fachmann hinlänglich bekannt und beispielsweise in S. Mitra, J. Am. Chem. Soc. 101 (1979) 3097 - 3110 beschrieben.

Die Aktivierung von Proteinen über die N-Hydroxysuccinimid-Funktion kann beispielsweise durch nucleophile Substitution an der ε-Aminogruppe der Lysinseitenkette von Proteinen im leicht alkalischen Puffer erfolgen. Hierzu wird ein 1- bis 15-facher Überschuß an Linker, bezogen auf die zu derivatisierenden Aminogruppen des Proteins, verwendet. Durch Dialyse oder Gelchromatographie werden das bei der Reaktion gebildete N-Hydroxysuccinimid und der überschüssige Linker abgetrennt.

Aminogruppenhaltige Haptene können entweder in organischen Medien, wie Dioxan oder DMF, unter Zusatz von Triethylamin, oder in Puffergemischen, bevorzugt Kaliumphosphatpuffer, pH 7,5, mit äquimolaren Mengen an erfindungsgemäß verwendeten Linkern umgesetzt werden.

Unter den drei Seitengruppen (Spacer), die den Linker darstellen, sind chemische Gruppen zu verstehen, deren Aufgabe die geeignete räumliche Anordnung von A, B und den Thioethergruppen ist und die die Wasserlöslichkeit der erfindungsgemäßen Konjugate gewährleisten.

Sonstige Zusammensetzung und Länge der Funktionen der homobidentalen trifunktionellen Linker sind unkritisch. Beispiele, wie derartige Linker aufgebaut sein können, sind in der EP-A 0 446 071 beschrieben, wobei die homobidentalen trifunktionellen Linker gemäß der Erfindung nicht wie die Linker gemäß der EP-A 0 446 071 drei Maleinimidofunktionen tragen dürfen, sondern lediglich zwei Maleinimidofunktionen und beispielsweise eine Bydroxysuccinimidfunktion bzw. zwei Thiolfunktionen und eine Hydroxysuccinimidfunktion. Weitere geeignete Linker sind beispielsweise in der EP-A 0 310 361 beschrieben, ebenfalls mit der Maßgabe, daß die Kopplungsgruppen homobidental aufgebaut sein müssen.

Analoges gilt für die in dem US-Patent 5,082,930 beschriebenen Linker.

Ebenfalls geeignete Linker sind beschrieben in der deutschen Patentanmeldung P 43 10 141.0 der Boehringer Mannheim GmbH, die mit gleichem Zeitrang wie die vorliegende Patentanmeldung zum Patent angemeldet wurde. Der Gegenstand dieser Patentanmeldung ist Gegenstand der Offenbarung der vorliegenden Patentanmeldung.

Bevorzugt ist es, als Spacergruppen im Linker gesättigte oder ungesättigte Alkylgruppen, die durch Imid-, Ether-, Carbonyl- oder Carboxygruppen unterbrochen sind, zu verwenden. Die Länge der drei Spacergruppen ist ebenfalls unkritisch und im wesentlichen dadurch bestimmt, daß keine sterischen Hinderungen zwischen A und B sowie bei der Bildung der Thioetherbindungen erfolgen soll. Zweckmäßigerweise enthalten die Spacergruppen zwischen 2 und 40 Atome.

Ein weiterer Gegenstand der Erfindung ist ein Verfahren zur Herstellung der erfindungsgemäßen Konjugate, welches darin besteht, daß in einem ersten Schritt eine erste zu koppelnde Substanz A mit einem homobidentalen trifunktionellen Linker L₁, der zwei Maleinimidogruppen enthält, gekoppelt wird, in einem zweiten Schritt eine zweite zu koppelnde Substanz B mit einem homobidentalen trifunktionellen Linker L₂, der zwei Thiolfunktionen enthält, gekoppelt wird und beide so aktivierten zu koppelnden Substanzen durch eine SH-Gruppen-Addition an Maleinimid über zwei Thioetherbindungen miteinander verknüpft werden.

Ein weiterer Gegenstand der Erfindung ist die Verwendung der erfindungsgemäßen Konjugate zur Bestimmung von immunologisch akiven Substanzen in immunologischen Tests.

Dabei können die erfindungsgemäßen Konjugate beispielsweise zur Vermittlung der Bindung des Analyten an eine Festphase oder zur Bindung des Analyten an eine Markierung (z. B. Enzyme) verwendet werden.

Besonders bevorzugt werden verwendet Antikörper/Enzymkonjugate, Antikörper/Biotinkonjugate, Bapten/Biotinkonjugate, Antikörper/Antikörper-Konjugate, Avidin/Streptavidin/Antikörperkonjugate, Avidin/Streptavidin-Haptenkonjugate.

Die Erfindung wird durch folgende Beispiele erläutert:

### Beispiel 1

### S,S-Diacetyl-6,8-dimercaptooktanoyl-bisphosphonat (Biphosphonat-LIPOS)

102.1 mg (3 mmol) Bisphosphonat werden in 7 ml Wasser gelöst und mit 155.8 mg (0.4 mmol) LIPOS (Beispiel 2) in 4 ml Dioxan versetzt. Der pH-Wert wird mit verdünntem Ammoniak auf pH 6-7 gestellt und 24 h bei 20°C gerührt. Anschließend werden weitere 78 mg (2 mmol) LIPOS in 2 ml Dioxan gelöst, nachdosiert; 24 h später gibt man nochmals 39 mg (0.1 mmol) LIPOS zu. Nach insgesamt 4 Tagen wird das Lösungsmittel im Wasserstrahlvakuum entfernt, der Rückstand zweimal mit je 15 ml THF digeriert und der Überstand abdekantiert. Das Rohprodukt wird in 0.5 ml Wasser gelöst, mit Methanol gefällt und im Exsiccator über CaCl₂ getrocknet.
- Ausbeute:: 20 mg (12 % d.Th.)
- DC:: Cellulose, n-Butanol/Eisessig/Wasser (v/v/v 3/1/1), Detektion mit Ninhydrin
Rf = 0.58
- 1H-NMR(D20/TMS-Na-Salz):: d = 1.30 - 2.00 (m, 10H, 5 CH₂); 2.23 (t, 2H, J=6.6 Hz, CH₂CO); 2.35 (s, 6H, CH₃-CO); 2.77 (s, 3H, N-CH₃); 2.77 - 3.70 ppm (m, 9H).

### Beispiel 2

### S,S-Diacetyl-6,8-dimercaptooktansäure-N-hydroxysuccinimidester (LIPOS)

### 2.1 Dihydroliponsäure

1.03 g (5 mmol) Liponsäure werden in 20 ml Wasser und 5 ml Ethanol gelöst, mit 380 mg (10 mmol) Natriumborhydrid versetzt und bei 20°C gerührt. Nach 60 min werden nochmals 380 mg (10 mmol) Natriumborhydrid zugegeben und weitere 5 h bei 20°C gerührt. Anschließend wird mit 50 ml Wasser verdünnt, mit 1 Salzsäure auf pH 1-2 gestellt und zweimal mit 50 ml Essigester extrahiert. Die vereinigten organischen Phasen über Natriumsulfat getrocknet, das Lösungsmittel im Wasserstrahlvakuum abdestilliert und der ölige Rückstand im Hochvakuum getrocknet.
- Ausbeute:: 1.0 g (95 % d.Th.)

### Analytische HPLC:

- Säule:: Vydac C18, 4.6 x 250 mm, 5 µm, 300 Å
- Laufmittel:: A: Millipore-Wasser, 0.01 % TFA
B: Acetonitril, 0.01 % TFA
- Gradient:: 0 - 65 % B in 90 min
- Fluß:: 1 ml/min
- Detektor:: UV-Detektion bei 226 nm
- Retentionszeit:: Dihydroliponsäure 46.7 min
Liponsäure 44.2 min
- DC:: Kieselgel (Merck 60), Isopropylether/1% Eisessig, Detektion mit N-Bromsuccinimid/Fluorescein-Spray Rf = 0.46 (Edukt Rf = 0.43)

### N-Bromsuccinimid/Fluorescein-Spray:

- Sprühlösung 1:: 0.2 % N-Bromsuccinimid in Methylenchlorid
- Sprühlösung 2:: 3 ml Fluorescein-Lösung (0.33 % Fluorescein in 0.1 N Natronlauge) mit 97 ml Ethanol verdünnt

### Detektion:

DC mit Lösung 1 besprühen, bei 20°C trocknen lassen, mit Lösung 2 nachsprühen.

### Färbung:

Nach längerem Stehen werden Schwefelverbindungen durch rosa Flecken auf gelbem Hintergrund sichtbar.

### 2.2 S,S-Diacetyl-6,8-dimercaptooktansäure

1 g (4.75 mmol) Dihydroliponsäure (Beispiel 2.1) werden in 50 ml Acetylchlorid aufgenommen und 15 h unter Rückfluß erhitzt. Anschließend wird die Reaktionslösung vorsichtig auf 500 ml Eiswasser gegossen und zweimal mit je 200 ml Essigester extrahiert. Die vereinigten organischen Phasen werden über Natriumsulfat getrocknet, das Lösungsmittel im Wasserstrahlvakuum abdestilliert und der Rückstand mittels offener Säulenchromatographie (Kieselgel Merck 60, 3 x 40 cm, Eluent: Ether/Hexan (v/v 4/1)/1 % Eisessig) aufgereinigt.
- Ausbeute:: 860 mg (62 % d.Th.)
- DC:: Kieselgel (Merck 60), Isopropylether/1% Eisessig, Detektion mit N-Bromsuccinimid/Fluorescein-Spray Rf = 0.45 (Edukt Rf = 0.43)

- HPLC:: Bedingungen siehe oben
- Retentionszeit:: 52.0 min (Di-Acetat)
49.8 min (Mono-Acetat)

- 1H-NMR (D₆-DMSO/TMS):: d = 1.44 (m, 6H); 1.60 (m, 2H); 2.19 (t, 2H, J=6.8 Hz, CH₂CO); 2.31 (s, 6H, 2 CH₃CO); 2.85 (m, 2H, CH₂-S); 3.40 ppm (m, 1H, CH-S).

### 2.3 S,S -Diacetyl-6,8-dimercaptooktansäure -N-hydrogysuccinimidester (LIPOS)

860 mg (2.94 mmol) Verbindung nach Beispiel 2.2 werden in 50 ml absoluten THF gelöst, mit 372 mg (3.23 mmol) N-Hydroxysuccinimid und 666 mg (3.23 mmol) Dicyclohexycarbodiimid versetzt. Nach 3 stündigem Rühren bei 20°C werden nochmals 115 mg (1 mmol) N-Hydroxysuccinimid und 206 mg (1 mmol) DCC zugegeben und weitere 24 h bei 20°C gerührt. Anschließend wird der ausgefallene Dicyclohexylharnstoff abfiltriert, das Filtrat eingeengt und in 20 ml absoluten THF aufgenommen. Unlösliches wird abfiltriert und das Lösungsmittel abdestilliert. Der Rückstand wird in 75 ml Essigester gelöst und zweimal mit je 40 ml Wasser extrahiert. Die Essigesterphase wird über Natriumsulfat getrocknet, das Lösungsmittel im Wasserstrahlvakuum entfernt und das Rohprodukt durch Chromatographie an Kieselgel (Merck 60, 4 x 30 cm, Eluent: Essigester/Hexan (v/v 3/2) aufgereinigt. Die gepoolten Fraktionen werden eingeengt und der Rückstand aus 20 ml Dioxan lyophilisiert.
- Ausbeute:: 820 mg (72 % d.Th.)
- DC:: Kieselgel (Merck 60), Ether/1 % Eisessig, Detektion mit N-Bromsuccinimid/Fluorescein-Spray.
Rf = 0.36
- 1H-NMR (D6-DMSO/TMS):: d = 1.30-1.89 (m, 8H); 2.31 (s, 6H, 2 CH₃CO); 2.59 (t, 2H, J=6.6 Hz, CH₂CO); 2.80 (s, 4H, 2 CH₂CO; 2.88 (m, 2H, CH₂-S); 3.40 ppm (m, 1H, CH-S).

### Beispiel 3

### 3.1 N-α-Boc-ε-maleinimido-α-aminohexansäure (α-Boc-ε-Mal-Lys)

3.70 g (15 mmol) α-N-t-Butyloxycarbonyl-Lysin werden in 200 ml ges. Natrium-hydrogen-carbonat-Lösung aufgenommen, mit 2.54 g (15 mmol) N-Ethoxycarbonyl-maleinimid versetzt und 30 min bei 20°C gerührt. Anschließend wird die Reaktionslösung mit 200 ml Wasser verdünnt, mit 2 N Salzsäure auf pH 1.8 gestellt, zweimal mit je 200 ml Essigester extrahiert, über Magnesiumsulfat getrocknet und das Lösungsmittel im Wasserstrahlvakuum abdestilliert. Das Rohprodukt wird mittels offener Säulenchromatographie (5 x 50 cm) über Kieselgel, Eluent Essigester/Methanol (v/v 4/1)/1 % Essigsäure, aufgereinigt. Die Fraktionen, die Produkt enthalten, werden vereinigt, das Lösungsmittel im Wasserstrahlvakuum entfernt und der Rückstand im Hochvakuum über CaCl₂ getrocknet.
- Ausbeute:: 3.8 g (11.58 mmol) 78 % d.Th.
- DC:: Kieselgel (Merck 60), Essigester/Methanol (v/v 3/2)/1% Essigsäure, Detektion mit Kaliumpermanganat R_{f} = 0.83
- ¹H-NMR (D₆-DMSO/TMS):: δ = 1.35 (m, 15H, ^{t}Bu u. 3 CH₂); 3.35 (m, 2H, CH₂-N); 3.66 (m, 1H, CH-N); 6.18 (d, br, 1H, NH, J=7.2 Hz); 6.98 ppm (s, 2H, CH=).

### 3.2 N-α-Boc-ε-maleinimido-α-aminohexanoyl-β-alanin (α-Boc-ε-Mal-Lys-β-Ala)

1.60 g (4.9 mmol) der Verbindung aus Beispiel 3.1 in 200 ml THF werden mit 676 mg (5.92 mmol) N-Hydroxysuccinimid und 1.21 g (5.92 mmol) Dicyclohexylcarbodiimid versetzt und 1 h bei 20°C gerührt. 480 mg (5.4 mmol) β-Alanin werden in 200 ml 0.1 M KPO₄-Puffer pH 8.5 gelöst und zum Reaktionsansatz getropft. Nach weiteren 16 h Rühren bei 20°C wird das organische Lösungsmittel im Wasserstrahlvakuum abdestilliert, die wässrige Phase mit 100 ml Wasser verdünnt, zweimal mit je 250 ml Essigester extrahiert, über Magnesiumsulfat getrocknet und im Wasserstrahlvakuum eingeengt. Der Rückstand wird über eine Kieselgelsäule (5 x 30 cm), Eluent Essigester/Methanol (v/v 4/1)/1 % Essigsäure aufgereinigt.
- Ausbeute:: 1.17 g (2.96 mmol) 61 % d.Th.
- DC:: Kieselgel (Merck 60), Essigester/Methanol (v/v 4/1)/1 % Eisessig, Detektion mit Kaliumpermanganat R_{f} = 0.7
- ¹H-NMR (D₆-DMSO/TMS):: δ = 1.35 (m, 15 H, ^{t}Bu u. 3 CH₂); 2.32 (t, 2H, CH₂CO, J=6.9 Hz); 3.16-3.41 (m, 4H, CH-N); 3.88 (m, 1H, CH-N); 6.73 (d, 1H, NH-COO, J=7.7 Hz); 6.98 (s, 2H, CH=); 7.78 ppm (t, 1H, NH-CO, J=5.4 Hz).

### 3.3 ε-Maleinimido-α-aminohexanoyl-β-alanin (ε-Mal-Lys-β-Ala)

710 mg (1.8 mmol) der Verbindung aus Beispiel 3.2 werden bei 0°C unter Rühren mit 15 ml Trifluoressigsäure versetzt, langsam auf 20°C erwärmt. Nach 30 min wird mit 15 ml Essigester verdünnt, weitere 15 min bei 20°C gerührt, das Lösungsmittel im Wasserstrahlvakuum abdestilliert und der Rückstand aus Dioxan/Wasser (v/v 1/1) lyophilisiert.
- Ausbeute:: 430 mg (1.5 mmol) 81 % d.Th.
- DC:: Kieselgel (Merck 60), Essigester/Methanol (v/v 1/4)/ 1 % Eisessig, Detektion mit Ninhydrin
R_{f} = 0.33
- ¹H-NMR (D₆-DMSO/TMS):: δ = 1.15-1.64 (m, 6H, 3 CH₂); 2.41 (t, 2H, CH₂-CO, J=6.6 Hz); 3.37 (m, 4H, 2 CH₂-N); 3.68 (m, 1H, CH-N); 7.00 (s, 2H, CH=); 8.53 ppm (t, 1H, NH-CO, J=6.0 Hz).

### 3.4 N-α-(6-Maleinimidohexanoyl)-ε-maleinimido-α-aminohexanoyl-β-alanin (α-MHS-e-Mal-Lys-β-Ala)

Zu einer Lösung aus 400 mg (1.35 mmol) der Verbindung aus Beispiel 3.3 in 10 ml 0.1 M KPO₄-Puffer pH 7.5 wird unter Rühren eine Lösung aus 462 mg (1.5 mmol) Maleinimidohexansäure-N-hydroxysuccinimidester (MHS) in 10 ml THF getropft. Der pH-Wert wird bis zur Konstanz mit 1 N Natronlauge auf pH 7.5 nachgestellt. Nach 16 stündigem Rühren bei 20°C wird das organische Lösungsmittel im Wasserstrahlvakuum abdestilliert, der Rückstand mit 10 ml Wasser verdünnt und mit 1 N Salzsäure auf pH 3.0 gestellt und dreimal mit je 30 ml Essigester extrahiert - das Produkt geht in die organische Phase über. Diese wird mit Magnesiumsulfat getrocknet und im Wasserstrahlvakuum eingeengt. Der Rückstand wird mittels offener Säulenchromatographie über Kieselgel, Eluent Essigester/Methanol(v/v 2/1)/1 % Essigsäure, gereinigt, die gepoolten Fraktionen eingedampft und aus 10 ml Essigester/THF (v/v 1/1) mit Diisopropylether ausgefällt. Der Niederschlag wird abfiltriert und im Hochvakuum über CaCl₂ getrocknet.
- Ausbeute:: 230 mg (0.47 mmol) 35 % d.Th.
- DC:: Kieselgel (Merck 60), Essigester/Methanol (v/v 2/1)/ 1 % Eisessig, Detektion mit
Kaliumpermanganat
R_{f} = 0.63
- ¹H-NMR (D₆-DMSO/TMS):: δ = 1.14-1.50 (m, 12H, 6 CH₂); 2.09 (t, 2H, CH₂-CO, J=6.6 Hz); 2.22 (t, 2H, CH₂-CO, J=7.5 Hz); 3.37 (m, 6H, CH₂-N); 4.07 (m, 1H, CH-N); 7.00 (s, 2H, CH=); 7.85 ppm (m, 2H, NH-CO).

### 3.5 N-α-(6-Maleinimidohexanoyl)-ε-maleinimido-α-aminohexanoyl-β-alanyl-(N-hydrogysuccinimid) (α-MHS-ε-Mal-Lys-β-Ala-OSu) (MALOS)

180 mg (0.36 mmol) der Verbindung aus Beispiel 3.4 werden in 10 ml absolutem DMF gelöst, mit 49 mg (0.43 mmol) N-Hydroxysuccinimid und 60 µl (0.43 mmol) Morpholinoethylisocyanat (MEI) versetzt und 16 h bei 20°C gerührt, wobei nach jeweils 8 h noch zweimal 49 mg (0.43 mmol) N-Hydroxysuccinimid und 60 µl (0.43 mmol) MEI nachdosiert werden. Zur vollständigen Umsetzung wird noch weitere 6 h gerührt. Anschließend wird das Lösungsmittel im Hochvakuum entfernt, der Rückstand mit 25 ml Essigester digeriert, Unlösliches abfiltriert und das Filtrat auf ca. 10 ml eingeengt. Das in wenig Essigester gelöste Produkt wird zu 200 ml Diisopropylether getropft, der Niederschlag abgetrennt (zum Absaugen nur Hausvakuum verwenden!), mit Diisopropylether gewaschen und im Trockenschrank mit CaCl₂ getrocknet.
- Ausbeute:: 115 mg (0.20 mmol) 56 % d.Th.
- DC:: Kieselgel (Merck 60), Essigester/Methanol (v/v 2/1)/ 1 % Eisessig, Detektion mit Kaliumpermanganat
R_{f} = 0.83
- ¹H-NMR (D₆-DMSO/TMS):: 6 = 1.06-1.50 (m, 12 H, 6 CH₂); 2.08 (t, 2H, CH₂CO, J=6.7 Hz); 2.50 (t, 2H, CH₂COOSu); 2.81 (s, 4H, 2 CH₂COO); 3.31 (m, 6H, 3 CH₂N); 4.12 (m, 1H, CH-NH); 6.98 (s, 4H, CH=); 7.81 (d, 1H, NHCH, J=7.0 Hz); 8.03 ppm (t, br, 1H, NHCH₂).

¹⁾Vorpolymerisierte POD (PODp) kann durch Reaktion von monomerer POD (PODₘₒₙₒ) mit Glutardialdehyd, wie bei Engvall und Perlmann (Immunochemistry 8 (1971) 871 - 874) beschrieben, hergestellt werden.

### Beispiel 4

### Aktivierung von PODp¹⁾ mit MALOS

Die Aktivierung erfolgt in einem molaren Überschuß von MALOS (Herstellung nach Beispiel 3.5) zu POD von 25 : 1. PODp wird mit einer Konzentration von 25 mg/ml in 50 mM Kaliumphosphatpuffer pH 7.5, 150 mM NaCl gelöst. Die entsprechende Menge MALOS wird in DMSO vorgelöst mit 100 mg/ml. Von dieser Lösung werden 92 µl/ml POD zur POD-Lösung gegeben. Der pH des Ansatzes wird nachkontrolliert und auf 7.0 gestellt. Anschließend wird der Ansatz unter Rühren 1 h/25°C inkubiert. Beendigung der Reaktion erfolgt durch pH-Änderung auf 6.1 und Abkühlung des Ansatzes im Eisbad. Anschließend wird das nicht gekoppelte MALOS durch fließende Dialyse gegen 10 mM Kaliumphosphatpuffer pH 6.1, 50 mM NaCl, 1 mM EDTA abgetrennt. Der Einbau der MH-Gruppen wird bestimmt durch Reaktion mit einer definierten Menge an Cystein und Titration der Restmenge an Cystein mit Dithiodipyridin.

### Beispiel 5

### Konjugation von PODp-MALOS mit Bisphosphonat-LIPOS

### 1. Abspaltung der Acetylschutzgruppe im Bisphosphonat-LIPOS

1 mg Bisphosphonat-LIPOS (Herstellung gem. Beispiel 1) wird in 1 ml 10 mM Kaliumphosphatpuffer pH 7.5, 50 mM NaCl, 2 mM EDTA gelöst. Von einer 1 M Hydroxylamin-Stammlösung werden 0.07 ml zugegeben (Endkonzentration 70 mM), der Ansatz wird 1 h/25°C unter Rühren inkubiert.

### 2. Kopplung mit PODp-MALOS

3 mg PODp-MALOS werden in 3 ml 10 mM Kaliumphosphatpuffer pH 7.5, 50 mM NaCl, 2 mM EDTA verdünnt. 0.044 ml des obigen Bydroxylaminolyse-Ansatzes werden zugegeben (entspricht einer Stöchiometrie von Bisphosphonat zu PODₘₒₙₒ von 1 : 1); der Ansatz wird 90 min./25°C unter Rühren inkubiert. Anschließend wird die Reaktion durch sukzessive Inkubation mit Cystein (ad 2 mM) und N-Ethyl-Maleinimid (ad 5 mM) beendet. Die Abtrennung der niedermolekularen Bestandteile erfolgt anschließend durch fließende Dialyse gegen 25 mM HEPES pH 6.8, 150 mM NaCl.

### Beispiel 6

### Aktivierung von Biotin mit MALOS

### 6.1. N-α-Boc-ε-Maleiuimido-α-aminohexanoyl-DADOO-Biotin

Zu einer Lösung aus 1.00 g (3.06 mmol) der nach Beispiel 3.1 hergestellten Verbindung in 50 ml THF werden unter Rühren 422 mg (3.67 mmol) N-Hydroxysuccinimid und 757 mg (3.67 mmol) Dicyclohexylcarbodiimid zugegeben. Nach 1 stündigem Rühren bei 20 °C tropft man eine Lösung aus 1.37 g (3.67 mmol) Biotin-DADOO in 50 ml 0.1 M KPO₄-Puffer pH 8.5 zu. Der Ansatz wird nach weitere 2 h bei 20 °C gerührt, anschließend das organische Lösungsmittel im Wasserstrahlvakuum entfernt und die wässrige Phase lyophilisiert. Das Rohprodukt wird durch Kieselgelchromatographie, Eluent Essigester/Methanol (v/v 2/1)/1 % Essigsäure, aufgereinigt.
- Ausbeute:: 930 mg (1.36 mmol) 44 % d.Th.
- DC:: Kieselgel (Merck 60), Essigester/Methanol (v/v 2/1)/1 % Eisessig
Detektion mit Biotinspray und Kaliumpermanganat
R_{f} = 0.44
- ¹H-NMR (D₆-DMSO/TMS):: d = 1.10-1.90 (m, 15H, ^{t}Bu u. 6 CH₂); 2.06 (t, 2H, CH₂CO, J=6.7 Hz); 2.60-2.90 (m, 3H, CHS u. CH₂S); 3.00-3.60 (m, 14H, 3 CH₂N u. 4 CH₂O); 3.77 (m, 1H, CHNH-Lys); 4.14 (dd, 1H, CH-NH-Biotin); 4.30 (dd, 1H, CHNH-Biotin); 6.39 (m, 2H, NH-Biotin); 6.76 (d, 1H, NHCOO, J=7.1); 6.99 (s, 2H, CH=); 7.84 ppm (t, br, 2H, 2 NHCO).

### 6.2 ε-Maleinimido-α-aminohexanoyl-DADOO-Biotin (ε-Mal-Lys-DADOO-Biotin)

500 mg (0.7 mmol) der nach Beispiel 6.1 hergestellten Verbindung werden bei 0°C mit 10 ml Trifluoressigsäure versetzt und langsam auf 20°C erwärmt. Nach 30 min wird mit 10 ml Essigester verdünnt und weitere 15 min gerührt. Das Lösungsmittel wird im Wasserstrahlvakuum entfernt und der Rückstand aus Dioxan/Wasser (v/v 1/1) lyophilisiert.
- Ausbeute:: ca. 0.7 mmol
- DC:: Kieselgel (Merck 60) Essigester/Methanol (v/v 1/1)/1 % Eisessig
Detektion mit Biotinspray, Kaliumpermanganat und Ninhydrin
R_{f} = 0.08

### 6.3 N-α-Maleinimidohexanoyl-ε-Maleinimido-α-aminohexanoyl-DADOO-Biotin (α-MHS-ε-Mal-Lys-DADOO-Biotin)

0.7 mmol der nach Beispiel 6.2 hergestellten Verbindung (enthält noch TFA) werden in 10 ml 0.1 M KPO₄-Puffer pH 7.5 gelöst und mit 260 mg (0.84 mmol) Maleinimidohexansäure-N-hydroxysuccinimid (MHS) in 10 ml THF versetzt. Der pH-Wert wird mit 1 N Natronlauge solange auf pH 7.5 nachgestellt bis keine pH-Wert-Änderung mehr zu beobachten ist. Nach 16 h Rühren bei 20 °C wird das organische Lösungsmittel im Wasserstrahlvakuum entfernt und der Rückstand lyophilisiert. Die Auf reinigung des Rohprodukts an Kieselgel, Eluent Essigester/Methanol (v/v 1/1)/1 % Essigsäure. Die gepoolten Fraktionen werden im Wasserstrahlvakuum eingeengt, der Rückstand in Dioxan aufgenommen, Ungelöstes abfiltriert und lyophilisiert.
- Ausbeute:: 500 mg (enthält noch Dioxan und Essigsäure)
- DC:: Kieselgel (Merck 60), Essigester/Methanol (v/v 1/1)/1 % Eisessig
Detektion mit Biotinspray und Kaliumpermanganat
R_{f} = 0.55
- ¹H-NMR (D₂O/TMS-Na-Salz):: d = 1.20-1.80 (m, 18 H, 9 CH₂); 2.22 (m, 4H, 2 CH₂CO); 2.71-2.90 (m, 3H, CHS u. CH₂S); 3.38-3.75 (m, 16 H, 4 CH₂N u. 4 CH₂O); 4.20 (m, 1H, CHNH-Lys); 4.44 (dd, 1H, CHNH-Biotin); 4.60 (dd, 1H, CHNH-Biotin); 6.88 ppm (s, 4H, CH=).

### Beispiel 7

### Lagerstabilität von IGG-(MB)-Biotin-(SH)-Konjugaten und von IGG-(MALOS)-Biotin-(LIPOS)-Konjugaten

### 7.1. Aktivierung von IGG mit MALOS (s. Beispiel 3.5.) und MHS (Maleinimidohexanoyl-N-hydroxy-succinimidester)

Jeweils 50 mg des IGG (es handelt sich um ein Maus-IGG aus Aszites, aufgereinigt über Ammoniumsulfatfällung und Anionenaustauschchromatografie) wurden parallel mit MALOS bzw. MHS umgesetzt. Die molare Stöchiometrie IGG : MALOS bzw. MHS war 1 : 25. MALOS und MHS wurden in DMSO vorgelöst; die Reaktion wurde in 50 mM Kaliumphosphatpuffer/150 mM NaCl, pH 7.5 durchgeführt. Die Konzentration des IGG war 25 mg/ml, Reaktionstemperatur 25°C, Reaktionszeit 1 h. Zum Stop der Reaktion wurde der pH des Reaktionsgemisches auf 6.1 heruntergesetzt und der Ansatz über Nacht bei 4°C dialysiert gegen 10 mM Kaliumphosphatpuffer, 50 mM NaCl, 1 mM EDTA, pH 6.5. Anschließend wurde nach bekannter Methode (Umsetzung der MH-Gruppen mit Dithiodipyridin) der Einbau der MH-Gruppen bestimmt.
- Ergebnis:: IGG-MALOS: 5,5 Mol MH/Mol IGG
IGG-MH: 5,5 Mol MH/Mol IGG

### 7.2. Biotinylierung des aktivierten IGG mit Biotin-LIPOS bzw. Biotin-C11-SH

Für die Biotinylierung des IGG-MALOS wurden zunächst die geschützten SH-Gruppen des Biotin-LIPOS durch Hydroxylaminolyse freigesetzt:
5 mg/ml Biotin-LIPOS wurden in 25 mM Hydroxylaminhaltigem Puffer (10 mM Kaliumphosphatpuffer, 50 mM NaCl, 2 mM EDTA, pH 7.5 1 h/25°C inkubiert. Danach wurde aus diesem Ansatz direkt die benötigte Menge Biotin-LIPOS für die Biotinylierung entnommen.

Für die Biotinylierung des IGG-MH wurde das Biotin-SH-Derivat direkt eingesetzt; dabei wurden alle verwendeten Puffer zuvor mit Stickstoff begast, um eine Oxidation der freien SH-Gruppen des Derivats möglichst zu verhindern. Beide Biotinylierungsansätze wurden in verschiedenen Stöchiometrien durchgeführt, jeweils 0.25 : 1, 0.5 : 1 und 1 : 1 (eingesetzte SH-Gruppen des Biotin-Derivats zu vorhandenen MH-Gruppen des IGG).
Die Reaktion erfolgte jeweils bei einer IGG-Konzentration von 1 mg/ml über 2 h bei 25°C in 10 mM Kaliumphosphatpuffer, 50 mM NaCl, 1 mM EDTA, pH 6,5. Die Reaktion wurde jeweils durch Zugabe von N-Methyl-Maleinimid (Endkonzentration 5 mM) gestoppt; anschließend wurden alle Ansätze fließend gegen 10 mM Kaliumphosphatpuffer, 50 mM NaCl, pH 7.5 dialysiert.
Nach der Dialyse wurde die Proteinkonzentration der Ansätze bestimmt. Jeder Ansatz wurde folgendermaßen behandelt:
Einstellen auf 0.8 mg/ml Protein in 50 mM Kaliumphosphatpuffer, 150 mM NaCl, pH 7.5; die Lösung wurde sterilfiltriert und bei 35°C zur Belastung eingelagert, bzw. bei -80°C zur Kontrolle eingefroren.

### 7.3. Überprüfen der Stabilität der Konjugate nach Belastung

Nach 2 Wochen wurde von allen Ansätzen der Biotin-Einbau und das freie Biotin im Ansatz bestimmt.
Der Biotin-Einbau wurde mit einem immunologischen Test folgendermaßen bestimmt:
Eine mit Streptavidin beschichtete Mikrotiterplatte wurde mit 100 µl IGG-Bi-Lösung pro Loch (200 ng/ml) 1 h inkubiert. Anschließend wurde nicht gebundenes IGG-Bi durch Waschen entfernt; das gebundene IGG-Bi wurde durch 1 h Inkubation mit Peroxidase-gekoppeltem Streptavidin und ABTS-Substrat nachgewiesen. Mit Hilfe einer mitgeführten Standardreihe von IGG mit bekanntem Biotinylierungsgrad konnte der Biotineinbau der Proben ermittelt werden.

Die Bestimmung des freien Biotins wurde mit einem immunologischen Test nach folgendem Prinzip durchgeführt: Biotin-beschichtete Röhrchen wurden mit Peroxidase-gekoppeltem Streptavidin inkubiert. Der Nachweis des gebundenen Streptavidins erfolgte durch Zugabe des POD-Substrats ABTS. Wurde zusammen mit der SA-POD die Probe mit freiem Biotin zugegeben, wurde das wandgebundene Streptavidin in Abhängigkeit vom Gehalt an freiem Biotin verdrängt (zunächst wurde aus allen Proben das biotinylierte IGG mit Trichloressigsäure ausgefällt; in den Test wurde dann der Überstand eingesetzt). Anhand einer Standardkurve mit freiem Biotin konnte dann der Biotin-Gehalt der Proben abgelesen werden.

Für die Bewertung der Lagerstabilität der verschiedenen Konjugate wurde zunächst der Biotineinbau in Mol Biotin pro Mol IGG berechnet, anschließend wurde das freie Biotin in Mol Biotin pro Mol IGG bestimmt. Schließlich wurde der prozentruale Ausbau an Biotin nach 2 Wochen Lagerung bei 35°C berechnet, bezogen auf den Biotin-Einbau der Lagerung bei -80°C.

Das Ergebnis zeigt die folgende Tabelle. Bei allen Stöchiometrien SB : MH waren die nach dem beanspruchten Verfahren hergestellten Konjugate deutlich, im Schnitt um Faktor 10 stabiler.

**Tabelle 1**

| Lagerstabilität der IGG-Bi-Konjugate | | | |
|---|---|---|---|
| Probe | Stöchiometrie SH : MH | Lagerform | prozentualer Ausbau von Biotin |
| IGG(MH)-Bi(SH) | 0.25 : 1 | -80°C | |
| IGG(MH)-Bi(SH) | 0.25 : 1 | +35°C | 4.1 % |
| IGG(MH)-Bi(SH) | 0.5 : 1 | -80°C | |
| IGG(MH)-Bi(SH) | 0.5 : 1 | +35°C | 6.9 % |
| IGG(MH)-Bi(SH) | 1 : 1 | -80°C | |
| IGG(MH)-Bi(SH) | 1 : 1 | +35°C | 5.6 % |
| | | | |
| IGG(MAL)-Bi(LIP) | 0.25 : 1 | -80°C | |
| IGG(MAL)-Bi(LIP) | 0.25 : 1 | +35°C | 0.3 % |
| IGG(MAL)-Bi(LIP) | 0.5 : 1 | -80°C | |
| IGG(MAL)-Bi(LIP) | 0.5 : 1 | +35°C | 0.5 % |
| IGG(MAL)-Bi(LIP) | 1 : 1 | -80°C | |
| IGG(MAL)-Bi(LIP) | 1 : 1 | +35°C | 0.7 % |

## Patentansprüche

1. Konjugate der allgemeinen Formel
A-L₁-L₂-B
in denen A und B zu koppelnde Substanzen ausgewählt aus der Gruppe der Haptene, Proteine, Peptide, Polysaccharide und anderer Polymere sind,
L₁ ein homobidentaler trifunktioneller Linker ist, der zwei Maleimidogruppen und eine Hydroxysuccinimid- oder Carbodiimidazolylgruppe enthält,
L₂ ein homobidentaler trifunktioneller Linker ist, der zwei Thiolfunktionen und eine Hydroxysuccinimid- oder Carbodiimidazolylgruppe enthält,
wobei die zu koppelnde Substanz A über die Hydroxysuccinimid- oder Carbodiimidazolylgruppe an L₁ und die zu koppelnde Substanz B über Hydroxysuccinimid- oder Carbodiimidazolylgruppe an L₂ gebunden ist, und die Linker L₁ und L₂ über die beiden Maleimidofunktionen von L₁ und die beiden Thiolfunktionen von L₂ über Thioetherbindungen miteinander verbunden sind.

2. Konjugate nach Anspruch 1 dadurch gekennzeichnet, daß A und B ausgewählt sind aus der Gruppe der Antikörper, Antikörperfragmente, Enzyme, Haptene, Biotin, Streptavidin und Peroxidase.

3. Verwendung von Konjugaten nach den Anprüchen 1 und 2 in einem immunologischen Test.

4. Verfahren zur Herstellung von Konjugaten nach den Ansprüchen 1 und 2, dadurch gekennzeichnet, daß in einem ersten Schritt eine erste zu koppelnde Substanz A mit einem homobidentalen, trifunktionellen Linker L₁, der zwei Maleinimidogruppen enthält, gekoppelt wird und in einem zweiten Schritt eine zweite zu koppelnde Substanz B mit einem homobidentalen, trifunktionellen Linker L₂, der zwei Thiolfunktionen enthält, gekoppelt wird und beide so aktivierten Substanzen AL₁ und BL₂ durch eine SH-Gruppen-Addition an Maleinimid über zwei Thioetherbindungen miteinander verknüpft werden.

5. Verfahren nach Anspruch 4, dadurch gekennzeichnet, daß die zu koppelnden Substanzen jeweils über eine Hydroxysuccinimid- oder Carbodiimidazolylfunktion an den homobidentalen, trifunktionellen Linker L₁ bzw. L₂ gekoppelt werden.

6. Verfahren nach den Ansprüchen 4 oder 5, dadurch gekennzeichnet, daß die zu koppelnden Substanzen ausgewählt sind aus der Gruppe Antikörper, Antikörperfragment, Enzym, Hapten.

7. Verfahren nach den Ansprüchen 4 - 6, dadurch gekennzeichnet, daß die zu koppelnden Substanzen ausgewählt sind aus der Gruppe bestehend aus: Biotin, Streptavidin und Peroxidase.

## Claims

1. Conjugates of the general formula
A-L₁-L₂-B
in which A and B are substances to be coupled, selected from the group comprising haptens, proteins, peptides, polysaccharides and other polymers,
L₁ is a homobidental trifunctional linker which contains two maleimido groups and a hydroxysuccinimide or carbodiimidazolyl group,
L₂ is a homobidental trifunctional linker which contains two thiol functional groups and a hydroxysuccinimide or carbodiimidazolyl group,
wherein the substance A to be coupled is bound to L₁ via the hydroxysuccinimide or carbodiimidazolyl group and the substance B to be coupled is bound to L₂ via the hydroxysuccinimide or carbodiimidazolyl group, and the linkers L₁ and L₂ are linked together by thioether bonds via the two maleimido functional groups of L₁ and the two thiol functional groups of L₂.

2. Conjugates as claimed in claim 1, wherein A and B are selected from the group comprising antibodies, antibody fragments, enzymes, haptens, biotin, streptavidin and peroxidase.

3. Use of conjugates as claimed in claims 1 and 2 in an immunological test.

4. Process for preparing conjugates as claimed in claims 1 and 2, wherein in a first step, a first substance A to be coupled is coupled to a homobidental, trifunctional linker L₁ containing two maleinimido groups and in a second step, a second substance B to be coupled is coupled to a homobidental, trifunctional linker L₂ containing two thiol groups and the two thus activated substances AL₁ and BL₂ are linked to each other via two thioether bonds by an SH group addition to maleinimide.

5. Process as claimed in claim 4, wherein the substances to be coupled are each coupled to the homobidental, trifunctional linkers L₁ and L₂ via a hydroxysuccinimide or carbodiimidazolyl group.

6. Process as claimed in claims 4 or 5, wherein the substances to be coupled are selected from the group comprising antibodies, antibody fragment, enzyme and hapten.

7. Process as claimed in claims 4 - 6, wherein the substances to be coupled are selected from the group comprising: biotin, streptavidin and peroxidase.

## Revendications

1. Conjugués de formule générale
A-L₁-L₂-B
dans lesquels A et B sont choisis, comme substances à coupler, dans le groupe des haptènes, protéines, peptides, polysaccharides et autres polymères,
L₁ est un linker trifonctionnel homobidenté, qui contient deux groupes maléimido et un groupe hydroxysuccinimide ou carbodiimidazolyle,
L₂ est un linker trifonctionnel homobidenté, qui contient deux fonctions thiol et un groupe hydroxysuccinimide ou carbodiimidazolyle,
dans lesquels la substance à coupler A est liée à L₁ par l'intermédiaire du groupe hydroxysuccinimide ou carbodiimidazolyle et la substance à coupler B est liée à L₂ par l'intermédiaire du groupe hydroxysuccinimide ou carbodiimidazolyle,
et les linkers L₁ et L₂ sont liés entre eux, par l'intermédiaire des deux fonctions maléimido de L₁ et des deux fonctions thiol de L₂, par des liaisons thioéther.

2. Conjugués selon la revendication 1, caractérisés par le fait que A et B sont choisis dans le groupe constitué par des anticorps, des fragments d'anticorps, des enzymes, des haptènes, la biotine, la streptavidine et des peroxydases.

3. Utilisation de conjugués selon les revendications 1 et 2, dans un test immunologique.

4. Procédé de préparation de conjugués selon les revendications 1 et 2, caractérisé par le fait que dans une première étape, une première substance à coupler A est couplée à un linker L₁ trifonctionnel homobidenté, qui contient deux groupes maléimido, et dans une deuxième étape, une deuxième substance à coupler B est couplée à un linker L₂ trifonctionnel homobidenté, qui contient deux fonctions thiol, et que les deux substances AL₁ et BL₂ ainsi activées sont reliées entre elles, par une addition de groupes SH sur le maléimide, par l'intermédiaire de deux liaisons thioéther.

5. Procédé selon la revendication 4, caractérisé par le fait que les substances à coupler sont couplées chacune, par l'intermédiaire d'une fonction hydroxysuccinimide ou carbodiimidazolyle, au linker L₁ ou L₂ trifonctionnel homobidenté.

6. Procédé selon la revendication 4 ou 5, caractérisé par le fait que les substances à coupler sont choisies dans le groupe constitué par des anticorps, des fragments d'anticorps, des enzymes, des haptènes.

7. Procédé selon les revendications 4 à 6, caractérisé par le fait que les substances à coupler sont choisies dans le groupe constitué par la biotine, la streptavidine et des peroxydases.
